(19) Europäisches Patentamt
European Patent Office
Office européen des brevets

(11) Veröffentlichungsnummer: **0 000 351**
**A1**

(12) # EUROPÄISCHE PATENTANMELDUNG

(21) Anmeldenummer: **78100270.4**

(22) Anmeldetag: **29.06.78**

(51) Int. Cl.2: **C07C149/32**, C07C149/40, A01N9/12

(30) Priorität: **07.07.77 CH 8426/77**
**27.01.78 CH 929/78**

(43) Veröffentlichungstag der Anmeldung: **24.01.79**
**Patentblatt 79/2**

(84) Benannte Vertragsstaaten: **BE CH DE FR GB NL**

(71) Anmelder: **CIBA-GEIGY AG, Patentabteilung Postfach, CH-4002 Basel (CH)**

(72) Erfinder: **Dürr, Dieter, Dr., Brändelistalweg 16, CH-4103 Bottmingen (CH)**
**Rohr, Otto, Dr., Kilbertweg 19, CH-4106 Therwil (CH)**
**Pissiotas, Georg, Dr., Breslauerstrasse 8, D-7850 Lörrach (DE)**
**Böhner, Beat, Dr., Hügelweg 3, CH-4102 Binningen (CH)**

(54) **Phenoxy-phenylthio-alkancarbonsäurederivate, Verfahren zu deren Herstellung und deren Verwendung als Herbizide und als Pflanzenwachstumsregulierungsmittel.**

(57) Die Erfindung betrifft neue Phenoxy-phenylthioalkan-carbonsäure-derivate mit herbizider und das Pflanzenwachstum regulierender, vor allem hemmender Wirkung entsprechend der Formel

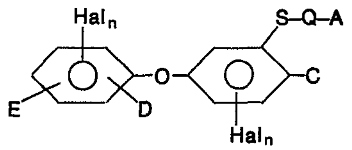

worin
A Cyan, Carboxyl, ein Carboxylsäuresalz, einen Ester, Thioester, ein Amid oder einen Imidoäther,
C Wasserstoff, Halogen, Cyan, $C_1$–$C_4$-Alkyl, $C_1$–$C_4$-Alkoxy, D und E dasselbe wie C, aber auch Nitro, Trifluormethyl, Methylsulfonyl oder Dimethylsulfonyl, «Hal» Halogen, n 0, 1 oder 2 bedeuten und Q eine Alkylenbrücke, die unsubstituiert oder durch Halogen, Alkoxy, Alkoxycarbonyl oder Cyan substituiert sein kann.

EP 0 000 351 A1

5-11560/=

BEZEICHNUNG GEÄNDERT
siehe Titelseite

Phenoxy-phenyl-thio-alkancarbonsäurederivate

Die vorliegende Erfindung betrifft neue Phenoxy-phenylthio-alkancarbonsäurederivate, welche herbizide und das Pflanzenwachstum regulierende Wirkung zeigen, Verfahren zur Herstellung dieser Derivate, herbizide und das Pflanzenwachstum regulierendes Mittel welche dieser Derivate als Wirkstoffe enthalten sowie die Verwendung der neuen Phenoxy-phenyl-thio-alkancarbonsäurederivate oder der sie enthaltenden Mittel als Herbizide und/oder zur Regulierung des Pflanzenwachstums.

Die neuen Phenoxy-phenylthio-alkancarbonsäurederivate entsprechen der Formel I

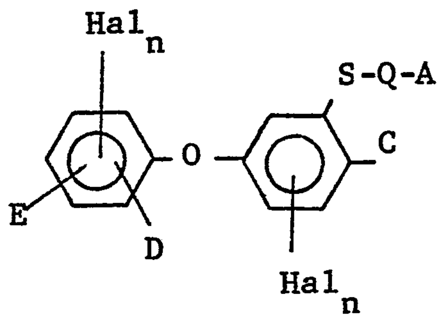

worin A die Cyanogruppe oder einen Rest - COB

B einen Rest $-O-N=CR_1R_2$, $-OR_3$, $-SR_4$ oder $-NR_5R_6$,

C Wasserstoff, Halogen, Cyan, $C_1$-$C_4$ Alkyl oder $C_1$-$C_4$ Alkoxy,

D Wasserstoff, Halogen, Cyan, Nitro, Trifluormethyl,

E Wasserstoff, Halogen, Cyan, Nitro, $C_1$-$C_4$ Alkyl, $C_1$-$C_4$ Alkoxy, Methylsulfonyl, Dimethylsulfamoyl,

Hal Halogen

n die Zahl 0, 1 oder 2,

Q eine verzweigte oder unverzweigte $C_1$-$C_4$ Alkylenbrücke, die unsubstituiert oder durch Halogen, $C_1$-$C_4$ Alkoxy, $C_1$-$C_4$ Alkoxycarbonyl oder Cyan substituiert sein kann,

$R_1$ und $R_2$ je $C_1$-$C_4$ Alkyl oder eines davon Wasserstoff,

$R_3$ und $R_4$ je Wasserstoff oder das Kation einer Base $\frac{1}{m} M^{m\oplus}$ bedeuten, wobei

M ein Alkali-, Erdalkali-Kation oder ein Fe, Cu-, Zn-, Mn-, Ni-Kation oder ein Ammonio-Rest

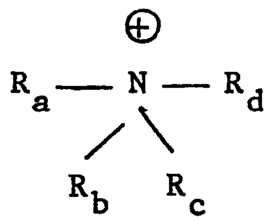

ist und

m als ganze Zahl 1, 2 oder 3 die Wertigkeit des Kations berücksichtigt, während $R_a$, $R_b$, $R_c$ und $R_d$ unabhängig voneinander Wasserstoff, Benzyl oder einen gegebenenfalls durch -OH, $-NH_2$ oder $C_1$-$C_4$ Alkoxy substituierten $C_1$-$C_4$ Alkylrest bedeuten,

$R_3$ und $R_4$ weiter einen $C_1$-$C_{18}$ Alkylrest, der unsubstituiert oder durch Halogen, $C_1$-$C_4$ Alkoxy, $C_1$-$C_4$ Alkylthio, $C_3$-$C_8$ Cycloalkyl, Nitro- oder Cyano substituiert sein kann, einen $C_3$-$C_{18}$ Alkenylrest, der unsubstituiert oder durch Halogen substituiert sein kann, einen $C_3$-$C_{18}$ Alkinylrest, einen Phenyl- oder Benzylrest der unsubstituiert oder durch Halogen, $C_1$-$C_4$ Alkyl, $C_1$-$C_4$ Alkoxy, $C_1$-$C_4$-Alkylthio, eine Cyano-, Nitro- oder Trifluormethylgruppe substituiert sein kann,

$R_5$ und $R_6$ unabhängig voneinander je Wasserstoff, $C_1$-$C_6$ Alkyl, das unsubstituiert oder $C_1$-$C_4$ Alkoxy, Halogen, Cyano, Hydroxyl oder $C_1$-$C_4$ Alkoxy substituiert sein kann oder

$R_5$ und $R_6$ zusammen mit dem Stickstoffatom, an das sie gebunden sind, einen 5-6 gliedrigen heterocyclischen Ring, der auch noch Stickstoff, Sauerstoff oder Schwefel als zweites Heteroatom enthalten kann und der durch $C_1$-$C_4$ Alkyl oder Phenyl substituiert sein kann.

Die Alkylreste in dieser Formel können verzweigt oder unverzweigt sein und enthalten die angegebene Anzahl Kohlenstoffatome.

Die Alkylenbrücke kann die Methylenbrücke, die 1- oder 2-Aethylenbrücke eine gerade oder als 1- oder 2-Methyläthyl oder 1,1-Dimethyl-methylen verzweigte Propylenbrücke, wie auch eine gerade oder entsprechend verzweigte Butylenbrücke sein.

Als 5-6 gliedrige Heterocyclen, die Reste $R_5$ und $R_6$ zusammen mit dem Stickstoffatom, an das sie gebunden sind bilden können, kommen die Pyrrol, Pyrrolidin, Pyridin und Piperidin in Frage, falls noch ein zweites Heteroatom im Ring ist, auch die Pyrazol-, Piperazin-, Oxazol-, Thiazol-, Morpholino-und Thiomorpholino-Ringe. Diese Ringe können auch durch $C_1$-$C_4$ Alkyl substituiert sein, der Piperazin-Ring auch durch Phenyl.

Die Verbindungen vorliegender Erfindung sind wenig giftig für Warmblüter und deren Applikation wirft keine Probleme auf. Sie sind stabile Verbindungen, welche in den üblichen organischen Lösungsmitteln, wie Alkoholen, Aethern, Ketonen, Dimethylformamid, Dimethylsulfoxid löslich sind. Normalerweise werden Aufwandmengen die zwischen 0,1 und 5 kg pro Hektar liegen benötigt.

Die neuen Wirkstoffe der Formel I gemäss vorliegender Erfindung besitzen Herbizidwirkung bei pre- und post-emergenter Anwendung und können in mono- und dikotylen Kulturen als Unkrautmittel eingesetzt werden.

Ferner besitzen sie günstige wachstumsregulierende Effekte (Wuchshemmung). Insbesondere hemmen sie das Wachstum von dicotylen Pflanzen. Beispiele für die nutzbringende Anwendung der erfindungsgemässen Verbindungen sind z.B.

- die Reduktion des vegetativen Wachstums bei Soja und ähnlichen Leguminosen, was zu einer Ertragssteigerung dieser Kultur führt;
- die Hemmung des unerwünschten Wachstums von Geiztrieben bei Tabak, dessen Haupttrieb man geschnitten hat, was der Ausbildung grössere und schönere Blätter zugute kommt;
- die Hemmung des Wachstums von Gras und dikotyledonen Pflanzen, wie Obstbäume, Zierbäume, Gebüsche und Hecken, zwecks Einsparung an Schnittarbeit.

Die Herstellung der neuen Verbindungen der Formel I erfolgt nach an sich bekannten Methoden und kann aus den folgenden Formelschema ersehen werden.

①

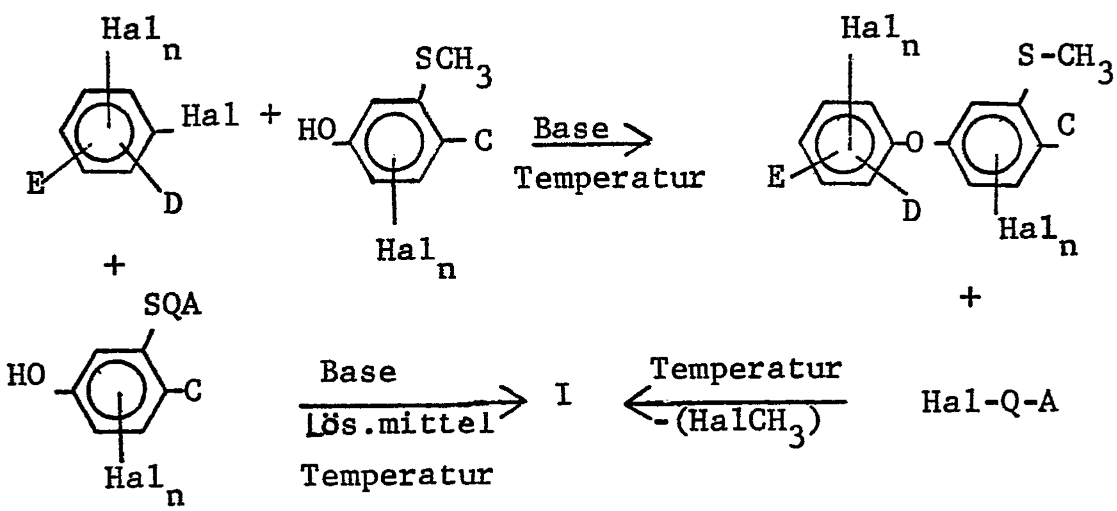


②

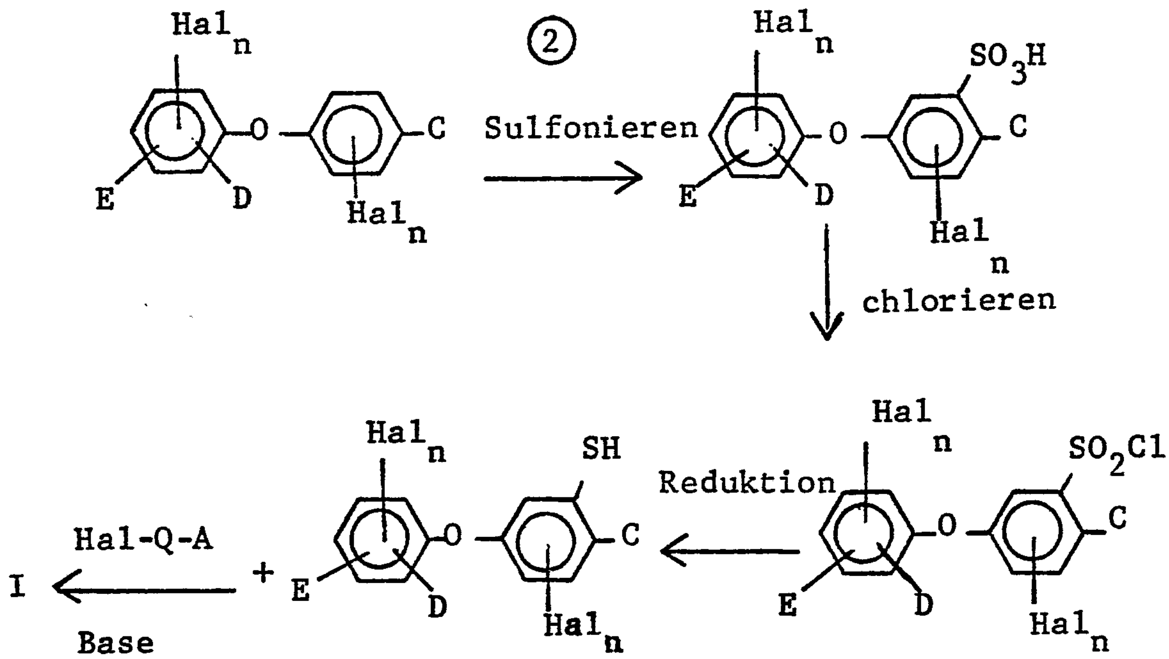

③

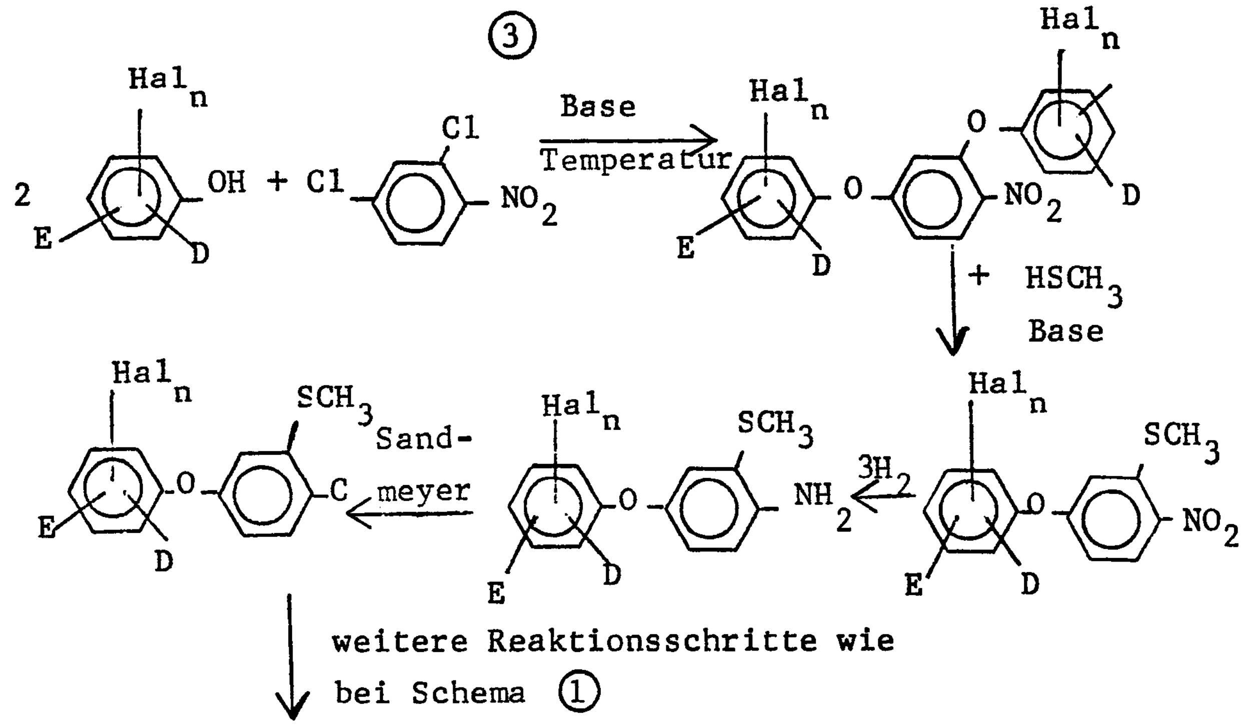
Base
Temperatur
+ $HSCH_3$
Base
$3H_2$
Sand-
meyer
weitere Reaktionsschritte wie
bei Schema ①

④

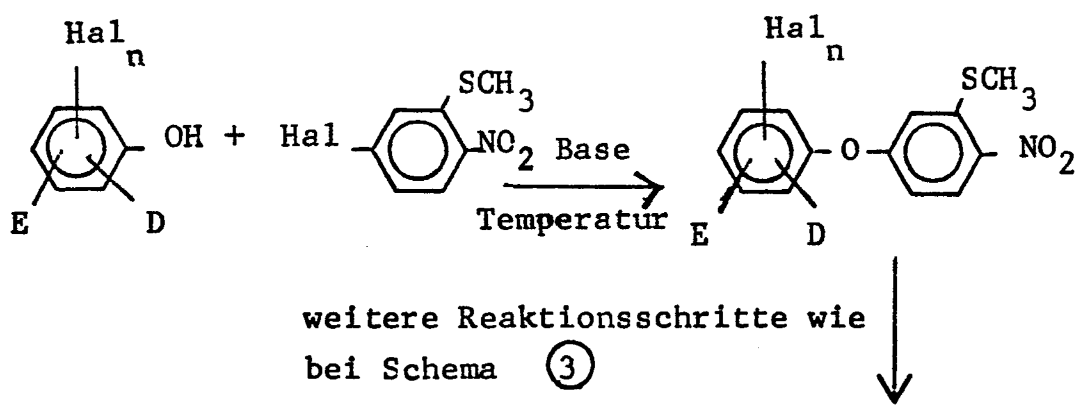
Base
Temperatur
weitere Reaktionsschritte wie
bei Schema ③

(5)

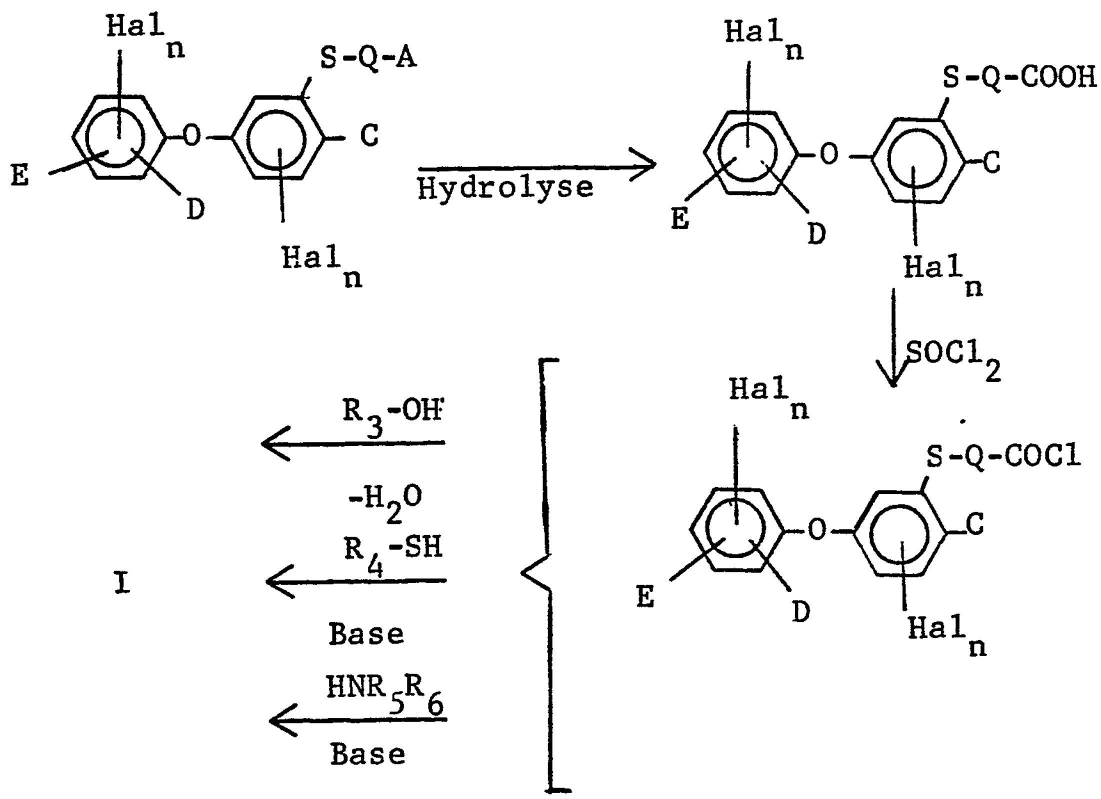


In diesen Formeln haben A, C, D, E, Hal, n und Q die unter Formel I gegebene Bedeutung.

Einzelne dieser Ausgangsverbindungen und deren Herstellung sind bekannt und beschrieben z.B. in den DOS 2 130 919 oder 2 333 848.

In den letzten Jahren ist eine reichhaltige Patentliteratur über herbizid wirksame, in verschiedener Weise substituierte Diphenyläther erschienen. Dabei sind vereinzelt auch substituierte Phenoxyphenylthio-alkancarbonsäurederivate bekanntgeworden, vgl. DOS 2 223 894.

Die herbizide Wirksamkeit der bekannten Phenoxyphenyl-thio-alkancarbonsäurederivate ist jedoch - insbesondere bei niedrigen Aufwandmengen - nicht befriedigend.

Es wurde nun überraschenderweise gefunden, dass die neuen Verbindungen der Formeln I starke herbizide Eigenschaften, insbesondere auch selektiv herbizide Wirksamkeit besitzen.

Die nachfolgenden Beispiele veranschaulichen diese Herstellungsverfahren näher. Weitere in entsprechender Weise hergestellte Wirkstoffe sind in den anschliessenden Tabellen aufgeführt. Temperaturangaben beziehen sich jeweils auf Celsius-Grade, Prozentangaben auf das Gewicht.

Beispiel 1

3-(2',4'-Dichlorphenoxy)-6-cyano-phenylthio-essigsäure-methylester

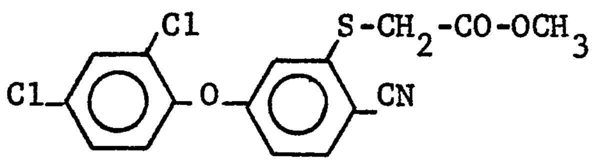

a) 12 g 4-Chlor-2-methylmerkapto-nitrobenzol werden in 50 ml Dimethylsulfoxyd mit 10 g 2,4-Dichlorphenol und 2,5 g Natriumhydroxyd auf 160° erhitzt. Nach zwei Stunden giesst man den Ansatz auf Eis und extrahiert mit Essigester.Die organische Lösung wird eingedampft und der Rückstand aus Toluol/Hexan umkristallisiert. Man erhält 16 g 2,4-Dichlor-3'-methylmerkapto-4'-nitrodiphenyläther vom Schmelzpunkt 120-122°. Die katalytische Reduktion in Dioxan mit Raney-Nickel ergibt daraus in nahezu quantitativer Ausbeute 2,4-Dichlor-3'-methylmerkapto-4'-amino-diphenyläther. Smp. 40-43°

b) 65 g dieser Verbindung werden mit 300 ml Wasser und 144 ml konz. Schwefelsäure vermischt und auf 0° gekühlt. Durch Umsatz mit einer 5%-iger Natriumnitritlösung bei 0-5° bereitet man eine Diazoniumsalzlösung, in die nach einer Stunde bei 0° eine Lösung

von 39,5 g Kaliumjodid in 370 ml Wasser eingetropft wird. Es bildet sich ein klebriger Niederschlag, der nach 2 Stunden durch Zugabe von Methylenchlorid gelöst wird. Die organische Phase wird abgetrennt und eingedampft. Der Rückstand wird mehrmals mit warmem Hexan extrahiert und die vereinigten Hexanextrakte eingeengt und abgekühlt. Durch Kristallisation erhält man 67 g 2,4-Dichlor-3'-methylmerkapto-4-jod-diphenyläther.

c) 15 g dieser Verbindung werden in 50 ml N-Methyl-2-pyrrolidon unter Stickstoff mit 3,5 g Kupfer-I-cyanid bei 140° verrührt. Nach 3 Stunden kühlt man ab und verrührt mit 500 ml Wasser und 20 ml konz. Ammoniak. Der Niederschlag wird gesammelt, getrocknet und aus Toluol/Hexan umkristallisiert. Man erhält 10 g 2,4-Dichlor-3'-methylmerkapto-4'-cyan-diphenyläther. Smp. 84°

d) 5 g dieser Verbindung werden in 20 ml Bromessigsäuremethylester am Rückfluss erhitzt. Nach 8 Stunden wird der Bromessigsäuremethylester abdestilliert und der Rückstand chromatographisch gereinigt. Man erhält neben unverändertem Ausgangsprodukt die Titelverbindung mit Schmelzpunkt 95°.

Beispiel 2

3-(2',4'-Dichlorphenoxy)-6-jod-phenylthioessigsäure-methylester

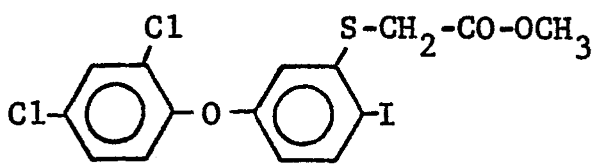

10 g 2,4-Dichlor-3'-methylmerkapto-4'-jod-diphenyläther wurden mit 20 ml Bromessigsäure-methylester 10 Stunden am Rückfluss erhitzt, die Reinigung erfolgte chromatographisch an einer Kieselsäuresäule mit Cyclohexan als Laufmittel. Man erhält nach Verdampfen des Lösungsmittels 7 g eines hellen Oeles mit dem Brechungsindex $n_D^{24}$ 1.6528.

Beispiel 3

β-[3-(2'-Chlor-4-trifluormethylphenyl)-phenylthio]-propionsäure-äthylester

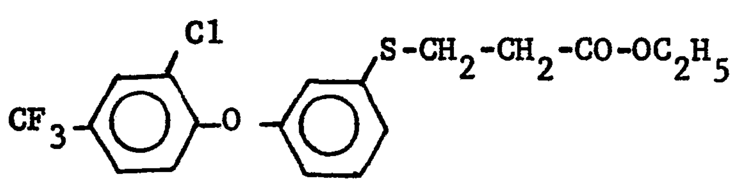

Man lässt 71 g 3-Methylmerkaptophenol, 118,5 g 3,4-Dichlorbenzotrifluorid und 22,5 g Natriumhydroxyd in 400 ml Dimethylsulfoxyd 4 Stunden bei 140° reagieren, giesst dann auf Eis, nimmt das sich ausscheidende Oel

in Aether auf und reinigt durch Destillation. 32 g dieses in guter Ausbeute erhaltenen 4-Trifluormethyl-2-chlor-3'-methylmerkapto-di-phenyläthers (Sdp. 115-120°/ 0,02 Torr) werden mit 90 g 3-Brompropionsäureäthylester und einem Kristall Kaliumjodid 10 Stunden bei 170° verrührt. Die Aufarbeitung erfolgt über eine Destillation und ergibt neben unverändertem Ausgangsprodukt die Titelverbindung. Siedepunkt 250° bei 0.03 Torr im Kugelrohr.

Beispiel 4

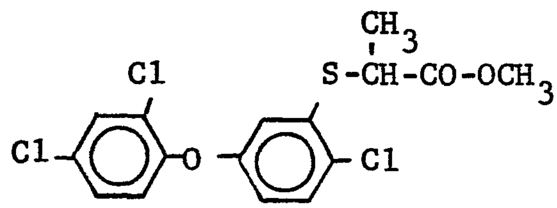

α-[3-(2',4'-Dichlorphenoxy)-6-chlor-phenylthio]-propionsäuremethylester

a) Eine Lösung von 400 g 2,4-Di-(2',4'-dichlorphenoxy)-nitrobenzol, 64 g 85%-igem Kaliumhydroxyd und 700 ml Dioxan wird mit 45 g Methylenmerkaptan bei 50-70° versetzt. Nach einer Stunde wird das Lösungsmittel am Vakuum abgezogen und der Rückstand mit Toluol aufgenommen. Die durch Waschen der Lösung mit verdünnter Natronlauge vom entstandenen 2,4-Dichlorphenol befreite Lösung wird zu einer siedenden Mischung von 500 g Eisenpulver, 500 g Alkohol und 50 ml konz. Salzsäure getropft. Nach 15 Stunden Rückfluss wird abgekühlt, alkalisch gemacht mit 30%-iger Natronlauge, vom Eisenlamm abgesaugt und der Filterkuchen mit Toluol gewaschen. Die organische Phase des Filtrats wird eingedampft und destilliert. Man erhält 192,5 g der Verbindung Beispiel 1a, Sdp. 170°/0,1

b) 180 g der Verbindung Beispiel 3a werden in 1,5 Liter Eisessig gelöst und mit 153 ml konz. Salzsäure versetzt. Durch Eintropfen einer Lösung von 41,5 g Natriumnitrit in 100 ml Wasser bei 5° wird eine Dixanlösung hergestellt, die nach dem Zerstören des Nitritüberschusses mit Sulfaminsäure bei 75-90° in 2 Liter einer 1°%-iger Salzsäure und 118 g Kupfer (I)chlorid getropft wird. Nach 1 Stunde extrahiert man mit Toluol, dampft die organische Phase ein und destilliert den Rückstand. Man erhält 172 g 3-(2',4'-Dichlorphenoxy)-6-chlormethylmerkaptobenzol Sdp. 158° /0,05 .

c) 140 g der Verbindung aus Beispiel 3b werden mit 105 ml 2-Brompropionsäuremethylester und einem Kristall Kaliumjodid 16 Stunden am Rückfluss gekocht. Die anschliessende Destillation ergibt 150 g der Titelverbindung. Sdp. 186°/0,09

Beispiel 5

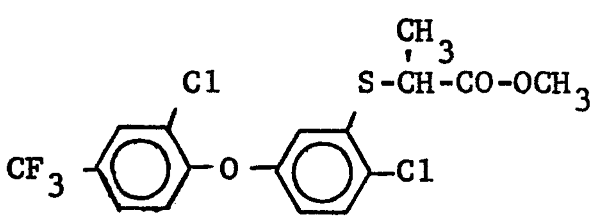

α-[3-(2'-Chlor-4'-trifluormethyl-phenoxy)-6-chlorthio]-propionsäuremethylester

a) 241 g 2-Chlor-5-methoxy-anilin werden unter starkem Rühren zu 310 ml Wasser und 385 ml konz. Salzsäure gegeben, gefolgt von 750 g Eis. Man kühlt die Mischung auf 10° und gibt auf ein Mal eine Lösung von 111 g

Natriumnitrit in 150 ml Wasser dazu, dabei steigt die Temperatur rasch auf 10°. Nach 30 Minuten wird der Nitritüberschuss mit Sulfaminsäure entfernt und die Lösung durch Filtration gelöst.

Diese Diazolösung wird bei 55-60° zu einer Lösung von 300 ml Wasser, 800 ml Toluol, 225 g Kaliummethylxanthogenat, 85 g Natriumcarbonat und 75 ml 30% Natronlauge getropft. Nach beendeter, sehr lebhafter Gasentwicklung trennt man die toluolische Phase ab. Diese wird mit Wasser gewaschen und nach dem Trocknen über Natriumsulfat in 100 ml Triäthylamin bei 70° getropft. 2 Stunden Rückfluss, dann abkühlen und Zugabe von 200 ml 30%-iger Natronlauge. Die organische Phase wird abgetrennt und destilliert. Man erhält 160 g 4-Chlor-3-methylmercaptoanisol. Sdp. 138-145°/11

152,5 g dieser Verbindung werden analog J.Am.Chem. Soc. 79,720 zu 110 g 4-Chlor-3-methylmerkaptophenol verseift. Sdp. 117-121°/0,03

b) 50 g 4-Chlor-3-methylmercaptophenol, 62 g 3,4-Dichlorbenzotrifluorid und 12 g Natriumhydroxyd werden in 150 ml Dimethylsulfoxyd 6 Stunden bei 142° verrührt. Dann giesst man das Reaktionsgemisch auf Eis, extrahiert mit Toluol und destilliert die organ. Phase. Man erhält 81 g 3-(2'-Chlor-4'-trifluormethyl-phenoxy)-6-chlor-thioanisol vom Sdp. 158°/0,4

c) 20 g dieser Verbindung aus Beispiel 4 b werden mit 30 ml 2-Brompropionsäuremethylester bei 190° 20 Stunden verrührt, dann erfolgt Destilliation im Kugelrohr. Man erhält 21 g der Titelverbindung. Sdp. 200°/0,13

Beispiel 6

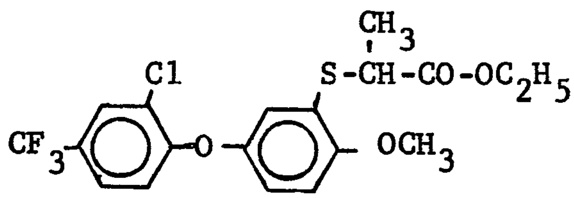

α-[3-(2'-Chlor-4'-trifluormethyl-phenoxy)-6-methoxy-thio]-propionsäureäthylester

a) 187 g Hydrochinonmonomethyläther, 324 g 3,4-dichlorbenzotrifluorid und 60 g Natriumhydroxyd werden in 500 ml Dimethylsulfoxyd 5 Stunden bei 140° verrührt. Man gibt die Reaktionslösung auf Eis und sammelt den gebildeten 2-Chlor-4-trifluormethyl-4'-methoxy-diphenyläther durch Nutschen. 315 g dieser Verbindung (Smp. 60-63°) werden in 1 Liter Eisessig mit 170 g einer Mischsäure aus 67 g rauchender Salpetersäure und 103 g konz. Schwefelsäure bei 10° tropfenweise versetzt. Man lässt noch 3 Stunden nachrühren, dann sammelt man den Niederchlag durch Nutschen. Man erhält nach dem Trocknen 291 g 4-(2'-Chlor-4-trifluormethyl-phenoxy)-2-nitro-anisol. Smp. 90-92°.

b) 318 g der Verbindung Beispiel 5a werden in Dioxan mit Nickel als Katalysator hydriert. Durch Abtrennen des Katalysators und Eindampfen der Reaktionslösung erhält man 300 g 4-(2'-Chlor-4'-trifluormethyl-phenoxy)-2-amino-anisol. Sdp. 185°/0,4 (Kugelrohr), Smp. 36-38°.

c) 97 g der Verbindung Beispiel 5b werden in 100 ml Dimethylformamid, 100 ml konz. Salzsäure und 100 ml Wasser mit Natriumnitrit nach Beispiel 4a diazotiert und mit Kaliummethylxanthogenat zu 4-(2'-Chlor-4'-trifluormethyl-phenoxy)-2-methylmercapto-anisol umgesetzt. Sdp. 150-155°/0,01

d) 8 g der Verbindung Beispiel 5c mit 2-Brompropionsäureäthylester analog Beispiel 3c umgesetzt ergeben die Titelverbindung. Sdp. 190°/0,001 (Kugelrohr )

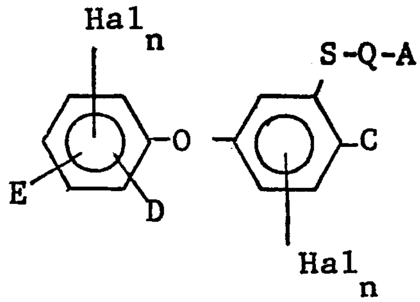

| No. | 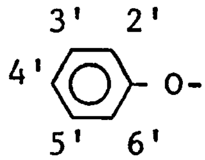 D, E, $Hal_n$ | 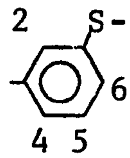 C, $Hal_n$ | -Q-A | physikalische Konstante (KR=Destillation im Kugelrohr) |
|---|---|---|---|---|
| 1 | 2'Cl 4'$CF_3$ | 6 Cl | $C_2H_5$<br>-CH-$COOC_2H_5$ | Sdp. 155°/ 0,001 KP |
| 2 | 2'CN 4'$CF_3$ | 6 Cl | $CH_3$<br>-CH-$COOCH_3$ | Sdp. 195°/ 0,001 KR |
| 3 | 2'Cl 4'$CF_3$ | 6 $OCH_3$ | $CH_3$<br>-CH-$COOC_8H_{37}$ | |
| 4 | 2'Cl 4'Cl | - | $CH_3$<br>-CH-$COOCH_3$ | |
| 5 | 2'Cl 4'Cl | 6 $CH_3$ | $CH_3$<br>-CH-$COOCH_3$ | |
| 6 | 2'Cl 4'Cl | 6 $OCH_3$ | $CH_3$<br>-CH-$COOCH_3$ | |
| 7 | 2'Cl 4'$CF_3$ | 6 $CH_3$ | $CH_3$<br>-CH-$COOCH_3$ | Sdp. 165°/ 0,001 |
| 8 | 2'Cl 4'$CF_3$ | 6 CN | $CH_3$<br>-CH-$COOCH_3$ | |
| 9 | 2'Cl 4'$CF_3$ | 6 Br | $CH_3$<br>-CH-CONH-$CH_2$-$CH_2$-$OCH_3$ | |

| No. | 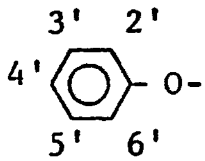 D, E, $Hal_n$ | 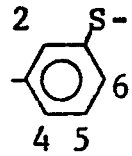 C, $Hal_n$ | -Q-A | physikalische Konstante |
|---|---|---|---|---|
| 10 | 2'Cl 4'$CF_3$ | 6 Cl | $-CH_2-COOCH_3$ | Sdp. 190°/ 0,05 KR |
| 11 | 2'$NO_2$ 4'$CF_3$ | - | $C_2H_5$<br>$-CH-COOCH_3$ | Sdp. 185°/0,04 KR |
| 12 | 4' $CF_3$ | - | $CH_3$<br>$-CH-COOC_2H_5$ | |
| 13 | 2'$NO_2$ 4'Cl | - | $-CH_2-CH_2-CO-N(C_2H_5)_2$ | |
| 14 | 2'$NO_2$ 4'Cl | 6 $OCH_3$ | $CH_3$<br>$-CH-COOCH_3$ | |
| 15 | 2'$CF_3$ 4'Cl | - | $C_2H_5$<br>$-CH-COO_nC_4H_9$ | |
| 16 | 2'$CF_3$ 4'$NO_2$ | 6 $OCH_3$ | $CH_3$ $CH_3$<br>$-CH-COO\ CH-COOCH_3$ | |
| 17 | 2'Cl 4'Cl | 6 CN | $-CH_2-COONa$ | |
| 18 | 2'Cl 4'Cl | 6 I | $-CH_2-COO\overset{\oplus}{N}H(CH_3)_3$ | |
| 19 | 2'Cl 4'Cl | 6 Cl | $-C_2H_4-COOCH_3$ | $n_D^{24}$ 1.6059 |
| 20 | 2'Cl 4'Cl | 6 Cl | $-CH_2-COOCH_3$ | Sdp. 230°/0,01KR |

| No. | 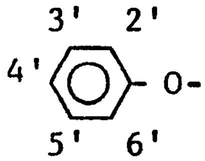 D, E, $Hal_n$ | 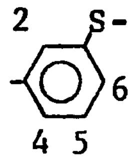 C, $Hal_n$ | -Q-A | physikalische Konstante |
|---|---|---|---|---|
| 21 | 2'Cl 4'Cl | 6 Cl | $-CH(CH_3)-COO-CH_2-CH_2-N(CH_3)-CH_3$ | |
| 22 | 2'Cl 4'Cl | - | $-C_2H_4COOCH_3$ | $n_p^{23}$ 1,5972 |
| 23 | 2'Cl 4'Cl | 6 I | $-CH(CH_3)-COOCH_3$ | $n_D^{23}$ 1,6185 |
| 24 | 2'Cl 4'$CF_3$ | - | $-CH(CH_3)-COOCH_3$ | Sdp. 140°/0,08 KR |
| 25 | 2'Cl 4'$CF_3$ | - | $-C_2H_4COO-CH_2-CO-CH_3$ | |
| 26 | 2'$NO_2$ 4'$CF_3$ | 6 Cl | $-CH_2-COOCH_3$ | Sdp. 235°/0,5 KR |
| 27 | 2'$NO_2$ 4'$CF_3$ | 6 Cl | $-CH(CH_3)-COOCH_3$ | Sdp. 235°/0,45 KR |
| 28 | 2'Cl 4'$CF_3$ | 6 Cl | $-CH(CH_3)-COO\overset{\oplus}{N}H_3-CH_2-CH_2-OH$ | |
| 29 | 2'Cl 4'$CF_3$ | 6 $OCH_3$ | $-CH(CH_3)-COOCH_3$ | Sdp. 195°/0,04 KR |
| 30 | 2'Cl 4'Cl | 6 Cl | $-CH(C_2H_5)-COOC_2H_5$ | Sdp. 185°/0,02 KR |

| No. | 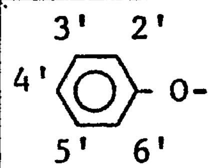<br>D, E, $Hal_n$ | 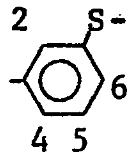<br>C, $Hal_n$ | -Q-A | physikalische Konstante |
|---|---|---|---|---|
| 31 | 2'Cl 4'Cl | 6 Cl | $-CH_2-CN$ | |
| 32 | 2'Cl 4'$CF_3$ | 6 Cl | $CH_3$<br>-CH-CN | |
| 33 | 2'Cl 4'$CF_3$ | 6 Cl | $-CH_2-CH_2-CN$ | |
| 34 | 2'Cl 4'Cl | 6 Cl | $-CH-CH_2-CH_2-CO-OCH_3$<br>$CH_3$ | |
| 35 | 2'Cl 4'Cl | 6 Cl | $CH_3$<br>-CH-CO-N<br>$OCH_3$<br>$CH_3$ | |
| 36 | 2'Cl 4 $CF_3$ | 6 $CH_3$ | $CH_3$<br>$-CH-CO-O-CH_2-CH_2-CN$<br>$CH_3$ | |
| 37 | 2'$CF_3$ 4'Cl | 6 Cl | $-CH-CO-S-CH_3$<br>$CH_3$ | |
| 38 | 2'Cl 4'Cl | 6 $CH_3$ | $-CH-CO-O-CH_2-CH_2-Cl$<br>$CH_3$ | |
| 39 | 2'$NO_2$ 4 Cl | 6 $OCH_3$ | $CH_3$<br>-CH-CO-O-N=C<br>$CH_3$<br>$CH_3$ | |
| 40 | 2'Cl,4'Cl,5'Cl | 6 Cl | $-CH-CO-O-CH_2-C\equiv CH$<br>$CH_3$ | |

| No. | 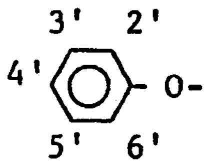<br>D, E, $Hal_n$ | 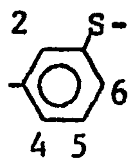<br>C, $Hal_n$ | -Q-A | physikalische Konstante |
|---|---|---|---|---|
| 41 | $2'Cl\ 4'CF_3$ | 6 CN | $-CH-CH_2-CO-O-$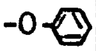<br>$CH_3$ | |
| 42 | $2'Cl\ 4'Cl,5'CH_3$ | 6 Br | $-CH-CO-O-NH_4^{\oplus}$<br>$CH_3$ | |
| 43 | $CH_3$<br>$2'Cl,4'SO_2N$<br>$CH_3$ | 4 Cl,6 Cl | $CH_3$<br>$-CH-CO-O-CH_2-C=CH_2$<br>$CH_3$ | |
| 44 | $2SO_2CH_3,4Cl$ | 6 $OCH_3$ | $-CH-CO-NH_2$<br>$CH_3$ | |
| 45 | 2Br, 4Br | 6 Cl | $-CH-CO-$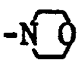<br>$CH_3$ | |

Die neuen Wirkstoffe der Formel I sind stabile Verbindungen, welche in üblichen organischen Lösungsmitteln, wie Alkoholen, Aethern, Ketonen, Dimethylformamid, Dimethylsulfoxyd etc. löslich sind.

Die Herstellung erfindungsgemässer Mittel erfolgt in an sich bekannter Weise durch inniges Vermischen und Vermahlen von Wirkstoffen der allgemeinen Formel I mit geeigneten Trägerstoffen und/oder Verteilungsmitteln, gegebenenfalls unter Zusatz von gegenüber den Wirkstoffen inerten Antischaum-, Netz-, Dispersions- und/ oder Lösungsmitteln, Die Wirkstoffe können in den folgenden Aufarbeitungsformen vorliegen und angewendet werden:

Feste Aufarbeitungsformen: Stäubemittel, Streumittel, Granulate, Umhüllungsgranulate, Imprägnierungsgranulate und Homogengranulate;

In Wasser Dispergierbare Wirkstoffkonzentrate: Spritzpulver (wettable powder), Paste, Emulsionen; Emulsionskonzentrate

Flüssige Aufarbeitungsformen: Lösungen

Die Wirkstoffkonzentrationen betragen in den erfindungsgemässen Mitteln 1 bis 80 Gewichtsprozent und können gegebenenfalls bei der Anwendung auch in geringen Konzentrationen wie etwa 0,05 bis 1% vorliegen.

Den beschriebenen erfindungsgemässen Mitteln lassen sich andere biozide Wirkstoffe oder Mittel beimischen.

Die folgenden Formulierungsbeispiele sollen die Herstellung von festen und flüssigen Aufbereitungsformen näher erläutern.

Emulsionskonzentrat

Zur Herstellung eines 25%igen Emulsionskonzentrates werden

25 Teile eines Wirkstoffes der Formel I,
5 Teile einer Mischung von Nonylphenolpolyoxyäthylen oder Calciumdodecylbenzolsulfat,
15 Teile Cyclohexanon,
55 Teile Xylol

miteinander vermischt. Dieses Konzentrat kann mit Wasser zu Emulsionen auf geeigneten Konzentrationen von z.B. 0,1 bis 10% verdünnt werden. Solche Emulsionen eigenen sich zur Bekämpfung von Unkräutern in Kulturpflanzungen.

Granulate

Zur Herstellung eines 5%igen Granulates werden die folgenden Stoffe verwendet:

| | | |
|---|---|---|
| 5 | Teile | eines der Wirkstoffe der Formel I, |
| 0,25 | Teile | Epichlorhydrin, |
| 0,25 | Teile | Cetylpolyglykoläther, |
| 3,50 | Teile | Polyäthylengylkol, |
| 91 | Teile | Kaolin (Korngrösse: 0,3-08 mm). |

Die Aktivsubstanz wird mit Epichlorhydrin vermischt und in 6 Teilen Aceton gelöst, hierauf wird Polyäthylenglykol und Cetylpolyglykoläther zugesetzt. Die so erhaltene Lösung wird auf Kaolin aufgesprüht und anschliessend im Vacuum verdampft.

Spritzpulver

Zur Herstellung eines a) 70%igen und b) 10%igen Spritzpulvers werden folgende Bestandteile verwendet:

| | | | |
|---|---|---|---|
| a) | 70 | Teile | eines Wirkstoffes der Formel I, |
| | 5 | Teile | Natriumdibutylnaphtylsulfonat, |
| | 3 | Teile | Naphthalinsulfonsäuren-Phenolsulfonsäuren-Formaldehyd-Kondensat 3:2:1, |
| | 10 | Teile | Kaolin, |
| | 12 | Teile | Champagne-Kreide; |
| b) | 10 | Teile | eines Wirkstoffes der Formel I, |
| | 3 | Teile | Gemisch der Natriumsalze von gesättigten Fettalkoholsulfaten, |
| | 5 | Teile | Naphthalinsulfonsäuren-Formaldehyd-Kondensat, |
| | 82 | Teile | Kaolin. |

Der angegebene Wirkstoff wird auf die entsprechenden Trägerstoffe (Kaolin und Kreide) aufgezogen und anschliessend mit den übrigen Bestandteilen vermischt und vermahlen. Man erhält Spritzpulver von vorzüglicher Benetzbarkeit und Schwebefähigkeit. Aus solchen Spritzpulvern können durch Verdünnen mit Wasser Suspensionen von 0,1-80% Wirkstoff erhalten werden, die sich zur Unkrautbekämpfung in Pflanzenkulturen eignen.

Paste

Zur Herstellung einer 45%igen Paste werden folgende Stoffe verwendet:

| | | |
|---|---|---|
| 45 | Teile | eines Wirkstoffes der Formel I, |
| 5 | Teile | Natriumaluminiumsilikat, |
| 14 | Teile | Cetylpolyglykoläther mit 8 Mol Aethylenoxid, |
| 1 | Teil | Oleylpolyglykoläther mit 5 Mol Aethylenoxid, |
| 2 | Teile | Spindeloel, |
| 10 | Teile | Polyäthynglykol, |
| 23 | Teile | Wasser. |

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Die neuen 3-Phenoxy-α-phenylthioalkancarbonsäurederivate der Formel I sowie die sie enthaltenden Mittel besitzen eine ausgezeichnete selektiv-herbizide Wirkung gegen Unkräuter in verschiedenen, vorzugsweise monokotylen Kulturpflanzenbeständen, ferner üben sie eine das Pflanzenwachstum regulierende Wirkung aus.

Ein besonders bevorzugtes Einsatzgebiet ist die selektive Bekämpfung von vor allem dikotylen Unkräutern in Getreidekulturen, speziell in Reis.

Obwohl die neuen Wirkstoffe der Formel I bei pre- und post-emergenter Anwendung wirksam sind, scheint die post-emergente Applikation als Kontaktherbizide den Vorzug zu verdienen, obwohl auch der pre-emergente Einsatz von Interesse ist.

Bevorzugt werden die neuen Wirkstoffe als z.B. 25%ige Spritzpulver oder z.B. 20%ige emulgierbare Konzentrate formuliert und mit Wasser verdünnt, auf die Pflanzenbestände post-emergent appliziert.

<u>Herbizide Wirkung bei Applikation der Wirkstoffe nach dem Auflaufen der Pflanzen (post-emergent.)</u>

Verschiedene Kulturpflanzen und Unkräuter werden aus den Samen in Töpfe im Gewächshaus aufgezogen, bis sie das 4- bis 6-Blatt-Stadium erreicht haben. Dann werden die Pflanzen mit wässerigen Wirkstoffemulsionen (erhalten aus einem 20%-igen emulgierbaren Konzentrat) in verschiedenen Dosierungen gespritzt. Die behandelten Pflanzen werden dann bei optimalen Bedingungen von Licht, Begiessung, Temperatur (22-25°C) und Luftfeuchtigkeit (50-70% relativ) gehalten. 15 Tage nach Behandlung werfolgte die Auswertung der Versuche.

In diesem Versuch hat die Verbindung No. 1 sämtliche getestete dikotylen Pflanzen und Unkräuter stark geschädigt. Die monokotylen Kulturen wurden grösstenteils geschont während die Ungräser nur leicht bis mittelstark geschädigt wurden.

Selektive herbizide Wirkung auf Reis im Nachauflaufverfahren

Reispflänzchen, welche 25 Tage alt sind, werden im Gewächshaus in grosse rechteckige Eternitschalen verpflanzt. Zwischen die Reihen der Reispflanzen werden dann Samen der in Reiskulturen vorkommenden Unkräuter Echinochloa crus galli, Cyperus difformis, Ammania und Rotala gesät. Die Schalen werden gut bewässert und bei einer Temperatur von ca. 25° und hoher Luftfeuchtigkeit gehalten. Nach 12 Tagen, wenn die Unkräuter aufgelaufen sind und das 2-3 Blattstadium erreicht haben, wird die Erde in der Schale mit einer 2,5 cm hohen Schicht Wasser bedeckt. Der Wirkstoff wird dann als Emulsionenskonzentrat mittels einer Pipette zwischen die Pflanzenreihen appliziert, wobei man das Emulsionskonzentrat so verdünnt und aufträgt, dass es einer Applikationsmenge im Feld von 2 und 1 kg Wirkstoff pro Hektar entspricht. Der Versuch wird nach 4 Wochen ausgewertet.

In diesem Versuch hat die Verbindung No. 1 die dikotylen Unkräuter Ammania und Rotala schwer sowie das Gras Cyperus difformis merklich geschädigt. Der Reis blieb ungeschädigt.

Die geprüften Verbindungen gemäss vorliegender Erfindung zeigten auf einigen Pflanzen ausgeprägte kontaktherbizide Wirkung und auf vielen Pflanzen Wachstumsstillstand als Symptom der wachstumshemmenden Eigenschaften.

Wuchshemmung bei Gräsern

In Kunststoffschalen mit Erde-Torf-Sand-Gemisch (6:3:1) wurden Samen der Gräser Lolium perenne, Poa pratensis, Festuca ovina und Dactylis glomerata ausgesät und normal bewässert. Die aufgelaufenen Gräser wurden wöchentlich bis auf 4 cm Höhe zurückgeschnitten und 40 Tage nach der Aussaat und 1 Tag nach dem letzten Schnitt mit wässerigen Spritzbrühen eines Wirkstoffs der Formel I bespritzt. Die Wirkstoffmenge betrug umgerechnet 5 kg Aktivsubstanz pro Hektar. 10 und 21 Tage nach Applikation wurde das Wachstum der Gräser beurteilt.

Wuchshemmung bei Getreide

In Kunststoffbechern wurde Sommerweizen (Triticum aestivum), Sommergerste (Hordeum vulgare) und Roggen (Secale) in sterilisierter Erde angesät und im Gewächshaus gezogen. Die Getreidesprösslinge werden 5 Tage nach Aussaat mit einer Spritzbrühe des Wirkstoffs behandelt. Die Blattapplikation entsprach 6 kg Wirkstoff pro Hektar. Die Auswertung erfolgt nach 21 Tage.

Die erfindungsgemässen Wirkstoffe bewirken eine merkliche Wuchshemmung sowohl bei den Gräsern wie beim Getreide.

## Patentansprüche

1. Phenoxy-phenylthio-alkancarbonsäurederivate der Formel I

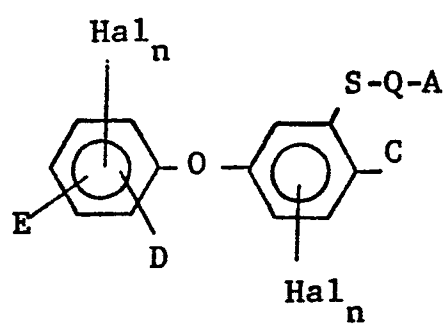

worin A die Cyanogruppe oder einen Rest - COB

B einen Rest $-O-N=CR_1R_2$, $-OR_3$, $-SR_4$ oder $-NR_5R_6$,

C Wasserstoff, Halogen, Cyan, $C_1$-$C_4$ Alkyl oder $C_1$-$C_4$ Alkoxy,

D Wasserstoff, Halogen, Cyan, Nitro, Trifluormethyl,

E Wasserstoff, Halogen, Cyan, Nitro, $C_1$-$C_4$ Alkyl, $C_1$-$C_4$ Alkoxy, Methylsulfonyl, Dimethylsulfamoyl,

Hal Halogen

n die Zahl O, 1 oder 2,

Q eine verzweigte oder unverzweigte $C_1$-$C_4$ Alkylenbrücke, die unsubstituiert oder durch Halogen, $C_1$-$C_4$ Alkoxy, $C_1$-$C_4$ Alkoxycarbonyl oder Cyan substituiert sein kann,

$R_1$ und $R_2$ je $C_1$-$C_4$ Alkyl oder eines davon Wasserstoff,

$R_3$ und $R_4$ je Wasserstoff oder das Kation einer Base $\frac{1}{m} M^{m\oplus}$ bedeuten, wobei

M ein Alkali-, Erdalkali-Kation oder ein Fe, Cu-, Zn-, Mn-, Ni-Kation oder ein Ammonio-Rest

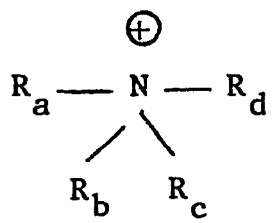

ist und

m als ganze Zahl 1, 2 oder 3 die Wertigkeit des Kations berücksichtigt, während $R_a$, $R_b$, $R_c$ und $R_d$ unabhängig voneinander Wasserstoff, Benzyl oder einen gegebenenfalls durch -OH, $-NH_2$ oder $C_1$-$C_4$ Alkoxy substituierten $C_1$-$C_4$ Alkylrest bedeuten,

$R_3$ und $R_4$ weiter einen $C_1$-$C_{18}$ Alkylrest, der unsubstituiert oder durch Halogen, $C_1$-$C_4$ Alkoxy, $C_1$-$C_4$ Alkylthio, $C_3$-$C_8$ Cycloalkyl, Nitro- oder Cyano substituiert sein kann, einen $C_3$-$C_{18}$ Alkenylrest, der unsubstituiert oder durch Halogen substituiert sein kann, einen $C_3$-$C_{18}$ Alkinylrest, einen Phenyl- oder

Benzylrest der unsubstituiert oder durch Halogen, $C_1$-$C_4$ Alkyl, $C_1$-$C_4$ Alkoxy, $C_1$-$C_4$-Alkylthio, eine Cyano-, Nitro- oder Trifluormethylgruppe substituiert sein kann,

$R_5$ und $R_6$ unabhängig voneinander je Wasserstoff, $C_1$-$C_6$ Alkyl, das unsubstituiert oder $C_1$-$C_4$ Alkoxy, Halogen, Cyano, Hydroxyl oder $C_1$-$C_4$ Alkoxy substituiert sein kann oder

$R_5$ und $R_6$ zusammen mit dem Stickstoffatom, an das sie gebunden sind, einen 5-6 gliedrigen heterocyclischen Ring, der auch noch Stickstoff, Sauerstoff oder Schwefel als zweites Heteroatom enthalten kann und der durch $C_1$-$C_4$ Alkyl oder Phenyl substituiert sein kann.

2. Die Verbindungen gemäss Patentanspruch 1 der Formel

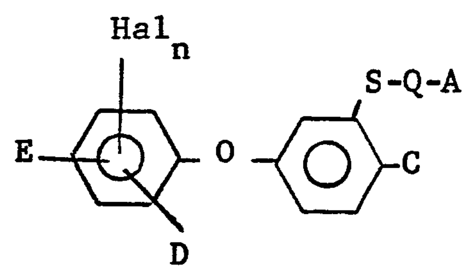

in denen C Wasserstoff, Halogen, Cyan, $C_1$-$C_4$ Alkyl oder $C_1$-$C_4$ Alkoxy,

D Wasserstoff, Halogen, Nitro oder Trifluormethyl,

E Wasserstoff, Halogen, Cyan oder Nitro,

Hal Halogen,

n 0, 1 oder 2,

Q ein verzweigtes oder unverzweigtes Alkylenbrückenglied mit 1-4 C-Atomen

bedeutet während A die unter Formel I, Anspruch 1 gegebene Bedeutung hat.

3. Die Verbindungen gemäss Patentanspruch 1 der Formel

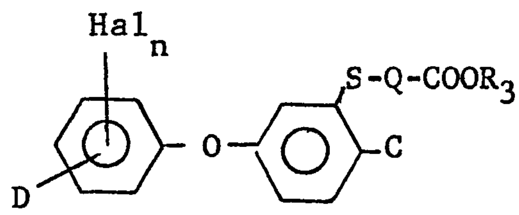

in denen C,D, Hal, n und $R_3$ die in Formel I, Anspruch 1 gegebene Bedeutung haben, während Q eine verzweigte oder unverzweigte $C_1$-$C_4$ Alkylenbrücke verkörpert.

4. Die Verbindungen gmäss Patentanspruch 1 der Formel

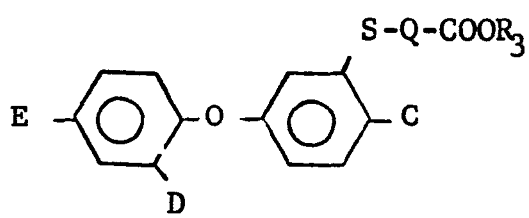

in denen C, D, E die unter Formel I, Anspruch 1 gegebene Bedeutung haben, während
Q eine verzweigte oder unverzweigte $C_1$-$C_4$ Alkylenbrücke und
$R_3$ Hydroxyl oder eine $C_1$-$C_4$ Alkoxygruppe verkörpert.

5. Die Verbindungen gemäss Patentanspruch 1 der Formel

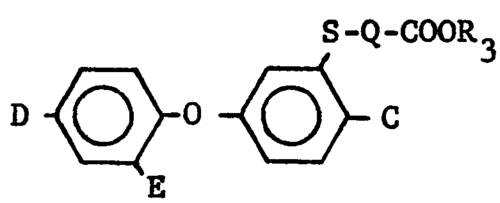

worin C, D, E, Q und $R_3$ die in Anspruch 4 gegebene Bedeutung haben.

6. Als Verbindung gemäss Anspruch 1 α-[3-(2',4'-Dichlorphenoxy)-6-chlorphenylthio]-propionsäure-methylester.

7. Als Verbindung gemäss Anspruch 1 α-[3-(2'-Chlor-4'-trifluormethylphenoxy)-6-chlorphenylthio]-propionsäure-methylester.

8. Als Verbindung gemäss Anspruch 1 α-[3-(2'-Chlor-4'-trifluormethoxy)-6-methoxyphenylthio]-propionsäure-methylester.

9. Als Verbindung gemäss Anspruch 1 3-(2',4'-Dichlorphenoxy)-6-cyanophenylthio-essigsäure-methylester.

10. Als Verbindung gemäss Anspruch 1 β-[3-(2',4'-Dichlorphenoxy)-6'-chlorphenylthio]-propionsäure-methylester.

11. Als Verbindung gemäss Anspruch 1 3-(2'-Nitro-4'-trifluormethylphenoxy)-6-chlorphenylthio-essigsäure-methylester.

12. Verfahren zur Herstellung der Phenoxy-phenylthioalkancarbonsäure-derivate der Formel I, Anspruch 1, dadurch gekennzeichnet, dass man in an sich bekannter Weise einen Alkylthio-diphenyläther der Formel

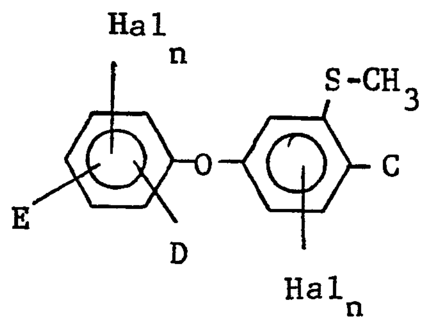

worin C, D, E, Hal und n die unter Formel I, Anspruch 1 gegebene Bedeutung haben, in einem aprotischen Lösungsmittel, in Gegenwart einer als Säurebinder eingesetzten Base, unter Normaldruck, bei einer Temperatur, die zwischen der Raumtemperatur und dem Siedepunkt des Reaktionsgemisches liegt, mit einem α-Halogencarbonsäurederivat der Formel

Hal - Q - A

worin Hal für ein Halogenatom, vorzugsweise Chlor- oder Brom steht während A und Q die unter Formel I, Anspruch 1 gegebene Bedeutung haben, umsetzt.

13. Herbizides und das Pflanzenwachstum regulierendes Mittel, dadurch gekennzeichnet, dass es als Wirkstoff mindestens ein Phenoxy-phenylthio-alkancarbonsäurederivat der Formel I, Anspruch 1 enthält.

Europäisches Patentamt

# EUROPÄISCHER RECHERCHENBERICHT

Nummer der Anmeldung

EP 78 10 0270

0000351

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | DE - A - 2 333 848 (BAYER AG)<br>* Seiten 38,44; Patentansprüche 1,4 * | 1,13 |
| D | DE - A - 2 223 894 (HOECHST)<br>* Seite 41; Patentanspruch 1 * | 1,13 |
| D | DE - A - 2 130 919 (NIPPON KAYAKU K.K.)<br>* Seiten 24,25; Patentansprüche 1,11 * | 1,13 |
| | FR - A - 2 325 638 (ISHIHARA SANGYO KAISHA)<br>* Seiten 36,37; Patentansprüche 1,8 * | 1,13 |
| A | FR - A - 2 285 861 (LABORATOIRE L.LAFON)<br>* Seite 52; Patentanspruch 1; Seite 15; Beispiel 10 * | 1 |
| A | CH - A - 424 761 (SANDOZ AG)<br>* Seite 5; Patentansprüche I,II * | 1,13 |

KLASSIFIKATION DER ANMELDUNG (Int.Cl.[2])

C 07 C 149/32
C 07 C 149/40
A 01 N 9/12

RECHERCHIERTE SACHGEBIETE (Int. Cl.[2])

C 07 C 149/32
C 07 C 149/40

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 21-09-1978 | BESLIER |